(19) Europäisches Patentamt
European Patent Office
Office européen des brevets

(11) **EP 4 336 516 A1**

(12) **EUROPEAN PATENT APPLICATION**

(43) Date of publication:
**13.03.2024 Bulletin 2024/11**

(21) Application number: **22194572.8**

(22) Date of filing: **08.09.2022**

(51) International Patent Classification (IPC):
**G16H 50/30** (2018.01)     **G16H 50/70** (2018.01)
**G16H 15/00** (2018.01)

(52) Cooperative Patent Classification (CPC):
**G16H 50/70; G16H 15/00; G16H 50/30**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **KACZMAREK, Piotr
  Eindhoven (NL)**
• **CHEUNG, Yee Him
  Eindhoven (NL)**
• **WU, Jie
  5656AG Eindhoven (NL)**

(74) Representative: **Philips Intellectual Property &
Standards
High Tech Campus 52
5656 AG Eindhoven (NL)**

(54) **METHODS AND SYSTEMS FOR VISUALIZING SURVIVAL STATISTICS FOR A PATIENT COHORT**

(57)     A method (100) for providing a visual representation of survival for a patient, comprising: (i) receiving (120) information about the patient, comprising at least a patient diagnosis; (ii) calculating (130), using the patient information and a reference survival dataset, one or more survival statistics for the patient for a first time period relative to a first historical cohort of patients; (iii) generating (140) a visual representation of the calculated one or more survival statistics; and (iv) providing (150), via a user interface of the system, the generated visual representation, wherein the visual representation comprises one or more of a survival function graph, a median survival graph, a mortality risk graph, and a survival probability graph.

FIG. 1

**Description**

FIELD OF THE INVENTION

The present disclosure is directed generally to methods and systems for generating and providing visual representations of survival for a patient over time.

BACKGROUND OF THE INVENTION

Visualization of data is an ever-growing and developing field. Visualization of medical data is one important part of that developing field, as proficient visualization can benefit many different aspects of medicine including diagnosis, treatment, and more. Indeed, visualization of medical data has become richer and more varied overtime due to the availability of electronic medical records (EMR). With the availability of EMR, for example, more patient information and larger patient cohorts are possible, resulting in much richer data analysis and visualization.

One important type of medical data is mortality data. For example, it is common to utilize mortality or survival of a patient cohort, given a common diagnosis of that patient cohort, over time. This provides an individual receiving that diagnosis with information about their mortality or survival odds for a similar time period. One common plot of survival is the Kaplan-Meier plot, which in the medical field graphs a calculation of the percentage of patients living for a certain amount of time after diagnosis or treatment. However, the Kaplan-Meier plot and visualizations like it are limited. They show data in only one form rather than encompassing the full array of information available from EMR.

SUMMARY OF THE INVENTION

Accordingly, there is a continued need for methods and systems for improved medical data visualization. Various embodiments and implementations herein are directed to a survival statistics analysis system that generated and provides a visual representation of survival for a patient. The survival statistics analysis system receives information about a patient, including at least a patient diagnosis and a date of diagnosis. The system calculates, using the patient information and a reference survival dataset, one or more survival statistics for the patient for a first time period relative to a first historical cohort of patients. The system then generates a visual representation of the calculated one or more survival statistics and provides the generated visual representation via a user interface of the system. According to an embodiment, the generated visual representation of survival for a patient comprises one or more of a survival function graph augmented with mortality data, a median survival graph, a mortality risk graph, and a survival probability graph.

Generally, in one aspect, a method for providing a visual representation of survival for a patient is provided. The method includes: (i) receiving information about the patient, comprising at least a patient diagnosis; (ii) calculating, using the patient information and a reference survival dataset, one or more survival statistics for the patient for a first time period relative to a first historical cohort of patients; (iii) generating a visual representation of the calculated one or more survival statistics; and (iv) providing, via a user interface of the system, the generated visual representation. According to an embodiment, the generated visual representation comprises one or more of:

1. a survival function graph, the survival function graph comprising a single graph with both: (i) a plot of a Kaplan-Meier estimate for the first historical cohort for the first time period; and (ii) a plot of trailing mortality, comprising a percentage of mortality of the first historical cohort for a trailing time period, the trailing time period being a windowed segment of the first time period;

2. a median survival graph, the median survival graph comprising a single graph with (i) an identification of a median survival time for the first historical cohort for the first time period; and (ii) a plot of a percentage of mortality of the first historical cohort for a pre-median survival time period; and (iii) a plot of a percentage of survival of the first historical cohort for a post-median survival time period;

3. a mortality risk graph, the mortality risk graph comprising a plot of trailing (also called moving-window) mortality, comprising a percentage of mortality, for a trailing time period, of the first historical cohort relative to a number of survivors of the first historical cohort at a beginning of the trailing time period, the trailing time period being a windowed segment of the first time period; and

4. a survival probability graph, the survival probability graph comprising a plot of survival probability over the first time period, wherein the survival probability is calculated for each of a plurality of time points over the first time period by dividing a total number of survivors of the first historical cohort at the end of the first time period by a total number of survivors of the first historical cohort at the time point being calculated.

According to an embodiment, the first time period is 5 years.
According to an embodiment, the trailing time period is 6 months.

According to an embodiment, the survival function graph further comprises a final survivor probability estimate for the first time period.

According to an embodiment, the median survival graph further comprises a final survivor probability estimate for the first time period.

According to an embodiment, providing the generated visual representation comprises providing two or more of: (i) the survival function graph; (ii) the median survival graph; (iii) the mortality risk graph; and (iv) the survival probability graph.

According to an embodiment, the user interface is configured to allow a user to navigate between the two or more graphs.

According to an embodiment, the user interface is configured to display two or more of the graphs at once.

According to an embodiment, each graph is calculated using each of a plurality of different historical cohorts of patients, each of the plurality of different historical cohorts of patients comprising a different relevant condition, such as tumor heterogeneity index, mutational burden, BMI, etc., for the patients in that respective historical cohort.

According to another aspect is a system for providing a visual representation of survival for a patient. The system includes: patient information comprising at least a patient diagnosis; a reference survival dataset; a processor configured to: (i) receiving information about the patient, comprising at least a patient diagnosis and a date of diagnosis; (ii) calculate, using the patient information and a reference survival dataset, one or more survival statistics for the patient for a first time period relative to a first historical cohort of patients; and (iii) generate a visual representation of the calculated one or more survival statistics; and a user interface configured to provide the generated visual representation. According to an embodiment, the generated visual representation comprises one or more of:

1. a survival function graph, the survival function graph comprising a single graph with both: (i) a plot of a Kaplan-Meier estimate for the first historical cohort for the first time period; and (ii) a plot of trailing mortality, comprising a percentage of mortality of the first historical cohort for a trailing time period, the trailing time period being a windowed segment of the first time period;

2. a median survival graph, the median survival graph comprising a single graph with (i) an identification of a median survival time for the first historical cohort for the first time period; and (ii) a plot of a percentage of mortality of the first historical cohort for a pre-median survival time period; and (iii) a plot of a percentage of survival of the first historical cohort for a post-median survival time period;

3. a mortality risk graph, the mortality risk graph comprising a plot of trailing mortality, comprising a percentage of mortality, for a trailing time period, of the first historical cohort relative to a number of survivors of the first historical cohort at a beginning of the trailing time period, the trailing time period being a windowed segment of the first time period; and

4. a survival probability graph, the survival probability graph comprising a plot of survival probability over the first time period, wherein the survival probability is calculated for each of a plurality of time points over the first time period by dividing a total number of survivors of the first historical cohort at the end of the first time period by a total number of survivors of the first historical cohort at the time point being calculated.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.

FIG. 1 is a flowchart of a method for generating and providing a visual representation of survival for a patient, in accordance with an embodiment.

FIG. 2 is a schematic representation of a survival statistics analysis system, in accordance with an embodiment.

FIG. 3A is an example of a survival function graph, in accordance with an embodiment.

FIG. 3B is an example of a survival function graph, in accordance with an embodiment.

FIG. 4A is an example of a median survival graph, in accordance with an embodiment.

FIG. 4B is an example of a median survival graph, in accordance with an embodiment.

FIG. 5A is an example of a mortality risk graph, in accordance with an embodiment.

FIG. 5B is an example of a mortality risk graph, in accordance with an embodiment.

FIG. 6A is an example of a survival probability graph, in accordance with an embodiment.

FIG. 6B is an example of a survival probability graph, in accordance with an embodiment.

FIG. 7 is an example of a survival function graph, in accordance with an embodiment.

FIG. 8 is an example of a median survival graph, in accordance with an embodiment.

FIG. 9 is an example of a mortality risk graph, in accordance with an embodiment.

FIG. 10 is an example of a survival probability graph, in accordance with an embodiment.

## DETAILED DESCRIPTION OF THE EMBODIMENTS

The present disclosure describes various embodiments of a system and method configured to generate and provide a visual representation of survival for a patient. More generally, Applicant has recognized and appreciated that it would be beneficial to provide improved methods and systems for the visualization of patient survival probability over time. Accordingly, a survival statistics analysis system is described which generates and provides a visual representation of survival for a patient. The survival statistics analysis system receives information about a patient, including at least a patient diagnosis and a date of diagnosis. The system calculates, using the patient information and a reference survival dataset, one or more survival statistics for the patient for a first time period relative to a first historical cohort of patients. The system then generates a visual representation of the calculated one or more survival statistics and provides the generated visual representation via a user interface of the system. According to an embodiment, the generated visual representation of survival for a patient comprises one or more of a survival function graph, a median survival graph, a mortality risk graph, and a survival probability graph.

It should be understood that while the systems and methods described or otherwise envisioned herein focus on the utilization of the system for survival statistics, solely for purposes of explanation. However, while survival data is used to illustrate the idea, the systems and methods applicable to any time-to-event data, where events may include, e.g., injury, onset of illness, recovery from illness, recurrence of a disease, discharge from hospital or failure of device or termination of relationship or attention, and a wide variety of other data. Typically, time-to-event data focus on the time elapsing before an event is experienced, often known as survival data in statistics. Notably, time-to-event data may be based on events other than death, such as recurrence of a disease event or discharge from hospital, among other events.

According to an embodiment, the systems and methods described or otherwise envisioned herein can, in some non-limiting embodiments, be implemented as an element for a commercial product for medical data such as Philips® IntelliSpace (available from Koninklijke Philips NV, the Netherlands), or as an element for a commercial product for patient analysis or monitoring, or any suitable system.

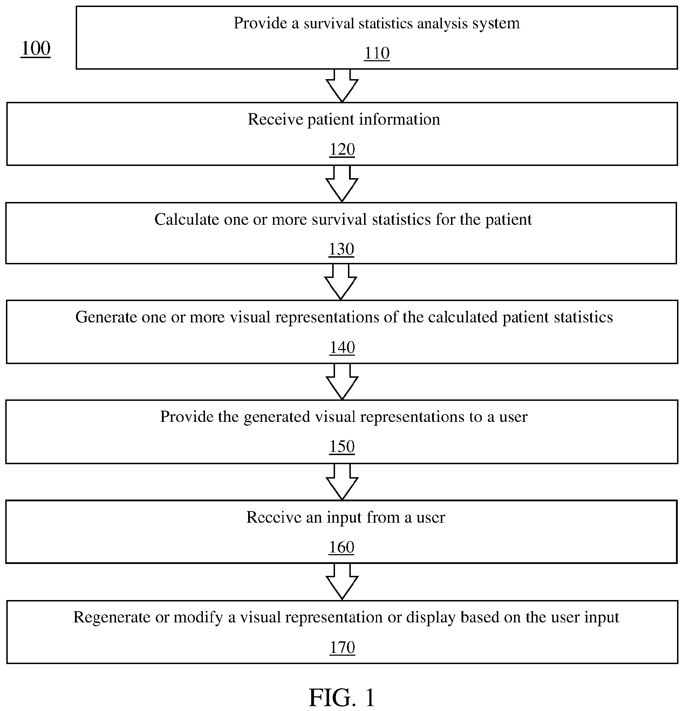

Referring to FIG. 1, in one embodiment is a flowchart of a method 100 for generating and providing a visual representation of survival for a patient using a survival statistics analysis system. The methods described in connection with the figures are provided as examples only, and shall be understood to not limit the scope of the disclosure. The survival statistics analysis system can be any of the systems described or otherwise envisioned herein. The survival statistics analysis system can be a single system or multiple different systems.

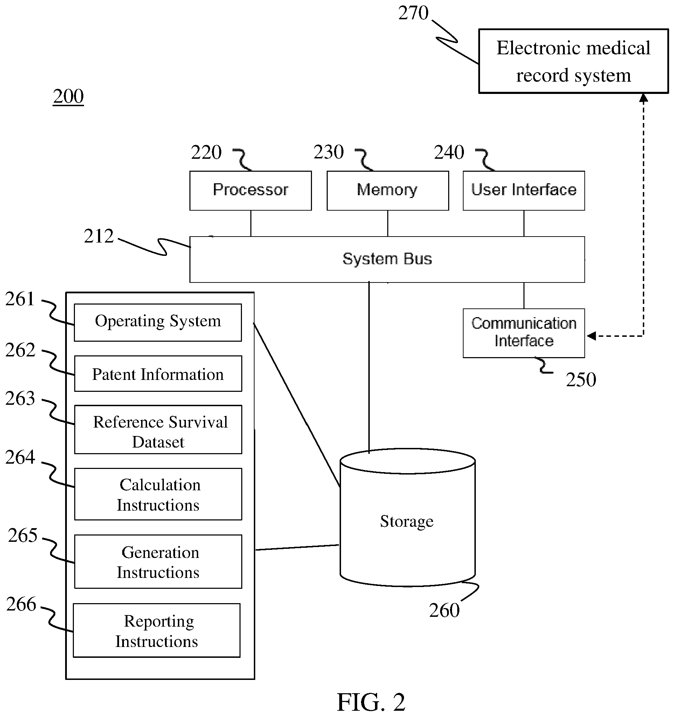

At step 110 of the method, a survival statistics analysis system 200 is provided. Referring to an embodiment of a survival statistics analysis system 200 as depicted in FIG. 2, for example, the system comprises one or more of a processor 220, memory 230, user interface 240, communications interface 250, and storage 260, interconnected via one or more system buses 212. It will be understood that FIG. 2 constitutes, in some respects, an abstraction and that the actual organization of the components of the system 200 may be different and more complex than illustrated. Additionally, survival statistics analysis system 200 can be any of the systems described or otherwise envisioned herein. Other elements and components of survival statistics analysis system 200 are disclosed and/or envisioned elsewhere herein.

At step 120 of the method, the survival statistics analysis system receives information about a patient. The patient information can be any information about the patient that the survival statistics analysis system can or may utilize for analysis as described or otherwise envisioned herein. According to an embodiment, the patient information comprises one or more of demographic information about the patient, a diagnosis for the patient, medical history of the patient, and/or any other information. For example, demographic information may comprise information about the patient such as name, age, body mass index (BMI), and any other demographic information. The diagnosis for the patient may be any information about a medical diagnosis for the patient, historical and/or current. The medical history of the patient may be any historical admittance or discharge information, historical treatment information, historical diagnosis information, historical exam or imaging information, and/or any other information.

The patient information is received from one or a plurality of different sources. According to an embodiment, the patient information is received from, retrieved from, or otherwise obtained from an electronic medical record (EMR) database or system 270. The EMR database or system may be local or remote. The EMR database or system may be a component of the survival statistics analysis system, or may be in local and/or remote communication with the survival statistics analysis system. The received patient information may be utilized immediately, or may be stored in local or remote storage for use in further steps of the method.

At step 130 of the method, the survival statistics analysis system calculates one or more survival statistics for the patient. Although the calculated output is identified herein as a "survival statistic," it will be understood that the term is non-limiting and means, among other possible definitions, a probability, likelihood, or estimate for the patient including mortality, survival, and/or many other probabilities, likelihoods, or estimates for the patient.

The system may calculate the one or more survival statistics for the patient on demand or as a result of programming or other automation. According to an embodiment, the survival statistics analysis system calculates one or more survival statistics for the patient in response to a user input. For example, a physician or other healthcare professional may request the information. Alternatively, the survival statistics analysis system may be partially or entirely automated, such that survival statistics for the patient are automatically calculated. For example, the system may be a standalone system or a component of a medical data or analysis system, which may comprise a user interface that displays information about a patient. This information may comprise one or more of the survival statistics and graphs described or otherwise envisioned herein.

The one or more survival statistics for the patient are calculated pursuant to any of the methods described or otherwise envisioned herein. For example, described below are several different non-limiting methods and examples for calculating the one or more survival statistics for the patient. These and many other methods may be utilized by the system.

According to an embodiment, the survival statistics analysis system calculates one or more survival statistics for the patient for a predetermined or user-defined first time period. The system may calculate the one or more survival statistics for any time period. Non-limiting examples of the first time period include days, weeks, months, years, and decades. For example, the system may calculate the one or more survival statistics for a time period of five years, which is a commonly-utilized time period for survival data, although the first time period may also be shorter or longer than five years.

According to an embodiment, the survival statistics analysis system comprises or is in communication with a reference survival dataset, which is utilized for the calculation of the one or more survival statistics for the patient. The reference survival dataset can be received from one or a plurality of different sources. According to an embodiment, the reference survival dataset is received from, retrieved from, or otherwise obtained from the electronic medical record (EMR) database or system 270. The EMR database or system may be local or remote. The EMR database or system may be a component of the survival statistics analysis system, or may be in local and/or remote communication with the survival statistics analysis system. Alternatively, the reference survival dataset may be a remote dataset, or another stored dataset of the system. The received reference survival dataset may be utilized immediately, or may be stored in local or remote storage for use in further steps of the method.

According to an embodiment, the reference survival dataset comprises information about one or more historical patients, which may be collected, filtered, or otherwise combined into one or more patient cohorts. A patient cohort may be, for example, a set of a plurality of historical patients for which survival data is available for at least the first time period. The patient cohort may be comprised of historical patients that have the same diagnosis, conditions, treatment, or other similarities to the patient for which the one or more survival statistics are being calculated. According to an embodiment, therefore, the system identifies a patient cohort relevant to a patient or subject being analyzed. According to another embodiment, the system identifies a patient cohort relevant to the patient or subject being analyzed, as well as to a patient cohort that does not have the same diagnosis, conditions, treatment, or other similarities to the patient for which the one or more survival statistics are being calculated. This later embodiment, for example, may enable analysis or determination of an impact of a diagnosis, conditions, treatment, or other similarities on the patient for which the one or more survival statistics are being calculated.

According to an embodiment, the calculated survival statistics for the patient are utilized immediately, or may be stored in local or remote storage for use in further steps of the method. Thus, for example, stored survival statistics for the patient can be recalled and utilized whenever a display is directed to, or is otherwise designed or programmed to, display patient information.

At step 140 of the method, the survival statistics analysis system generates one or more visual representations of the calculated one or more survival statistics. This may be performed immediately after the one or more survival statistics are calculated, or may be performed using stored survival statistics. The system may generate the one or more visual representations of the calculated one or more survival statistics on demand or as a result of programming or other automation. According to an embodiment, the survival statistics analysis system generates the one or more visual representations in response to a user input. For example, a physician or other healthcare professional may request the information. Alternatively, the survival statistics analysis system may be partially or entirely automated, such that the one or more visual representations are automatically calculated. For example, the system may be a standalone system or a component of a medical data or analysis system, which may comprise a user interface that displays information about a patient. This information may comprise one or more of the survival statistics and graphs described or otherwise envisioned herein.

The one or more visual representations of the calculated one or more survival statistics are generated pursuant to any of the methods described or otherwise envisioned herein. For example, described below are several different non-limiting methods and examples of visual representations of calculated survival statistics, and thus the system may be designed or programmed to generate such visual representations using known methods and mechanisms for graphing data. These and many other methods may be utilized by the system.

Once generated, the one or more visual representations of the calculated one or more survival statistics may be utilized immediately, or may be stored in local or remote storage for use in further steps of the method.

At step 150 of the method, the survival statistics analysis system provides, via a user interface, the generated one or more visual representations of the calculated one or more survival statistics. For example, a visual representation may be displayed to a medical professional or other user, including the patient, via the user interface of the system. The generated one or more visual representations may be provided to a user via any mechanism for display, visualization, or otherwise providing information via a user interface. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands. As just one non-limiting example, the user interface may be a component of a patient monitoring system.

According to an embodiment, the display may further comprise patient information such as demographic information about the patient, a diagnosis for the patient, medical history of the patient, and/or any other information.

According to an embodiment, as described herein, some or all of the displayed information may be manipulatable, in response to user input provided via the user interface.

The survival statistics analysis system provides, via a user interface, a generated visual representation of the calculated one or more survival statistics, which can be any of a variety of different statistics and visual representations. According to one embodiment, the visual representation comprises a survival function graph. The survival function graph can include a single graph that displays two different survival statistics, preferably overlaid. The first survival statistic of the survival function graph is a plot of a Kaplan-Meier estimate for the first historical cohort for the first time period. A Kaplan-Meier estimate is a statistic used to estimate a survival function over time. In the healthcare setting, the Kaplan-Meier estimate can be a measure of the fraction of patients or subjects living for a certain amount of time after diagnosis or after treatment. According to an embodiment, the Kaplan-Meier estimate plot in the graph is based on integrated data from multiple Kaplan-Meier type estimators. The survival function may present overall survival data, disease-free survival, or progression-free survival, among other options. The second survival statistic of the survival function graph is a visualization of mortality in the same period, comprising a plot of trailing mortality. This trailing mortality comprises a percentage of mortality of the first historical cohort for a trailing time period, where the trailing time period is a windowed segment of the first time period. The trailing time period can be any time period which is a subset or segment of the first time period. For example, the trailing time period can be weeks, months, or years. According to one embodiment, the trailing time period is six months, although it may be shorter or longer. According to another embodiment, the trailing time period is one year, although it may be shorter or longer. The trailing time period may be predetermined or preprogrammed, or may be determined in whole or in part based on user input received via the user interface.

According to an embodiment, the first historical cohort for this graph, and any of the other graphs, comprises a set of a plurality of historical patients for which survival data is available for at least the first time period, which is collected, filtered, or otherwise retrieved from the reference survival dataset. For example, the first historical cohort may be comprised of historical patients that have the same diagnosis, conditions, treatment, or other similarities to the patient for which the one or more survival statistics are being calculated. As described herein, the reference survival dataset - and therefore the first historical cohort - can be received or generated from one or a plurality of different sources, and can be a component of the survival statistics analysis system, or may be in local and/or remote communication with the survival statistics analysis system, such as a remote dataset, or another stored dataset of the system.

According to an embodiment, the first time period for this graph, and any of the other graphs, comprises any time period for which the survival statistics are desired. According to another embodiment, the time period is five years, although it may be shorter or longer. The time period may be predetermined or preprogrammed, or may be determined in whole or in part based on user input received via the user interface.

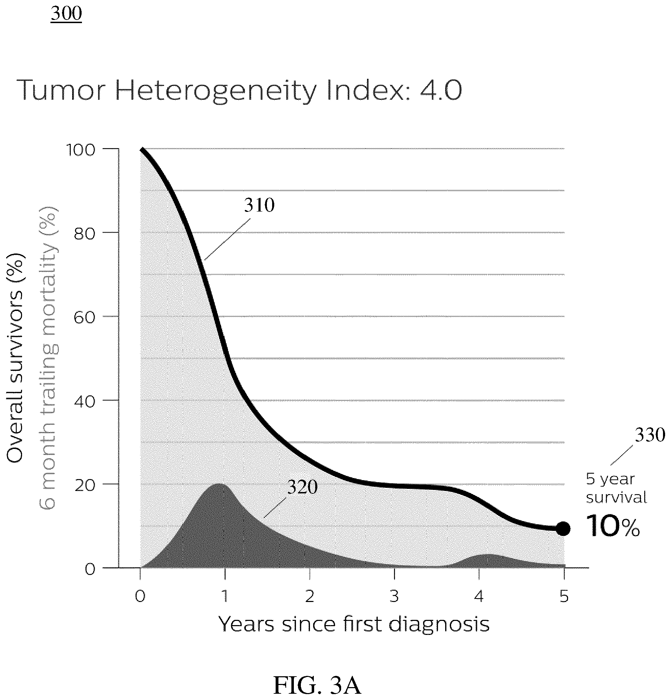

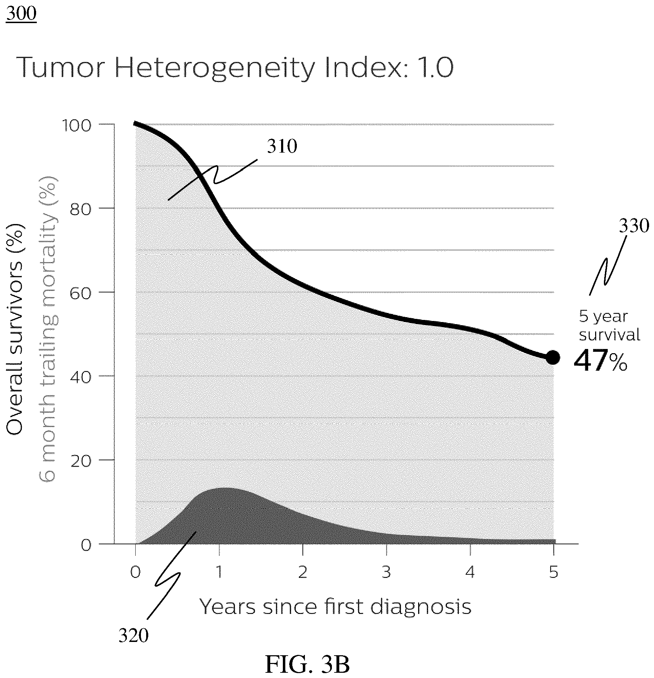

Referring to FIGS. 3A and 3B are survival function graphs 300. Each survival function graph comprises survival statistics for five years since initial diagnosis, although as described herein this is a non-limiting option. The timeframe may be longer or shorter, and the starting point may be initial diagnosis, treatment, or any other determined starting event. Each survival function graph comprises a Kaplan-Meier estimate 310 and a trailing mortality plot 320. To emphasize the survival chances for the entire time period (in this case five years) the last point on the survival function is a final survival percentage 330, which in this example is a 5-year survival percentage of 10%.

Referring to FIG. 3A, the graph comprises, in this embodiment, a first historical cohort with a particular determined tumor heterogeneity index of 4.0, which indicates a higher level of heterogeneity of cells within the sampled tumor (compared to an index of 1.0, 2.0, 3.0, and variations thereof, for example). In this example, the higher heterogeneity index is correlated with lower survival odds including a 5-year survival percentage of 10%. Referring to FIG. 3B, the graph comprises, in this embodiment, a first historical cohort with a particular determined tumor heterogeneity index of 1.0, which indicates a lower level of heterogeneity of cells within the sampled tumor. In this example, the higher heterogeneity index is correlated with higher survival odds including a 5-year survival percentage of 47%.

According to another embodiment, the visual representation comprises a median survival graph. The median survival graph can include a single graph that displays several different pieces of information, preferably overlaid. The first information provided by the graph is an identification of a median survival time for the first historical cohort for the first time period. The second piece of information is a plot of a percentage of mortality of the first historical cohort for a pre-median survival time period, which will be displayed to the left side of the median survival time. The third piece of information is a plot of a percentage of survival of the first historical cohort for a post-median survival time period, which will be displayed to the right side of the median survival time.

According to an embodiment, this novel method of representing survival time focuses attention on median survival time. For an untrained user, such as a patient, this approach helps recognize the cohort's average life expectancy and how likely it is for death to occur around group average time.

According to an embodiment, the graph is split into two distinct sections using different functions for the period before and after the median survival time (MST). In the interval preceding MST chart tracks the mortality rate as a percentage of overall cohort. In the period following MST the graph uses survival function - number of survivors as a percentage of overall cohort. This type of compound visual concentrates attention on the average survival time. Second important benefit of this approach is the clear depiction of the probability of decease around MST represented by steepness of 'median peak'.

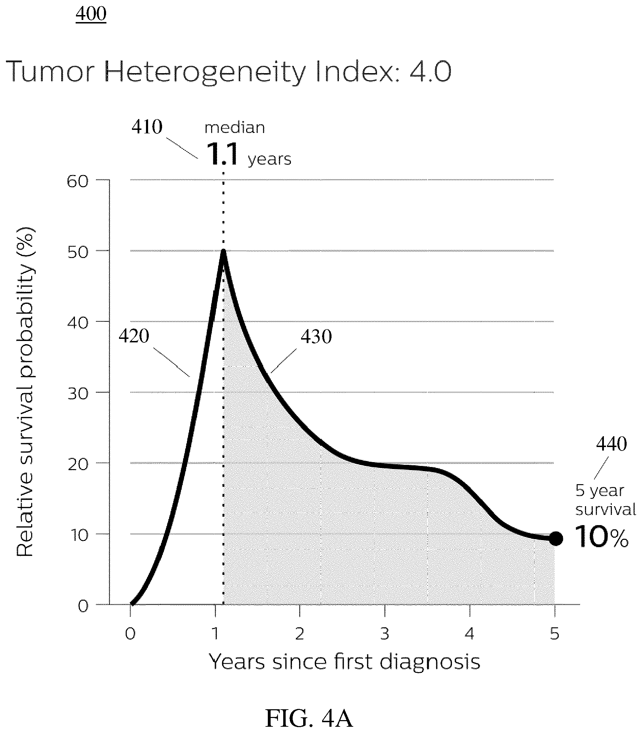

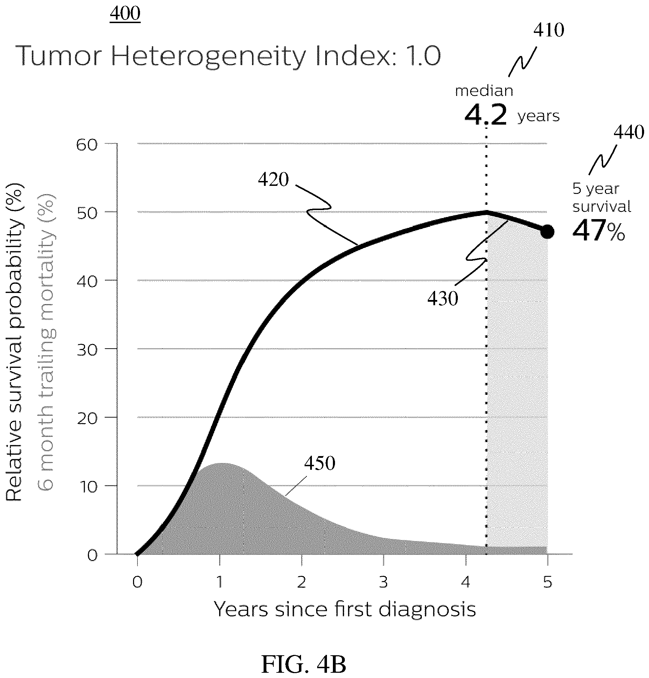

Referring to FIGS. 4A and 4B are median survival graphs 400. Each median survival graph comprises survival statistics for five years since initial diagnosis, although as described herein this is a non-limiting option. The timeframe may be longer or shorter, and the starting point may be initial diagnosis, treatment, or any other determined starting event. Each median survival graph comprises a median survival time 410, a pre-median plot of the mortality rate as a percentage of overall cohort 420, and a post-median plot of the number of survivors as a percentage of overall cohort 430. To emphasize the survival chances for the entire time period (in this case five years) the last point on the survival function is a final survival percentage 440, which in this example is a 5-year survival percentage of 10%. The graphs illustrate how deaths can be concentrated in the period around MST giving the average number more significance (THI = 4.0 graph in FIG. 4A) or more spread out (THI = 1.0 in FIG. 4B) diminishing its predictive value.

Referring to FIG. 4B, to provide additional information the visual may be layered with a mortality graph 450 showing mortality in a trailing period as the percentage of the original cohort. For example, the trailing period in this graph is 6 months, although the trailing period may be shorter or longer.

Referring to FIG. 4A, the graph comprises, in this embodiment, a first historical cohort with a particular determined tumor heterogeneity index of 4.0, which indicates a higher level of heterogeneity of cells within the sampled tumor. In this example, the higher heterogeneity index is correlated with lower survival odds including a 5-year survival percentage of 10%. Referring to FIG. 4B, the graph comprises, in this embodiment, a first historical cohort with a particular determined tumor heterogeneity index of 1.0, which indicates a lower level of heterogeneity of cells within the sampled tumor. In this example, the higher heterogeneity index is correlated with higher survival odds including a 5-year survival percentage of 47.

According to another embodiment, the visual representation comprises a mortality risk graph. The mortality risk graph can include a single graph that displays a plot of trailing mortality. The plot of trailing mortality comprises a percentage of mortality, for a trailing time period, of the first historical cohort relative to a number of survivors of the first historical cohort at a beginning of the trailing time period, where the trailing time period is a windowed segment of the first time period. The trailing time period can be any time period which is a subset or segment of the first time period. For example, the trailing time period can be weeks, months, or years. According to one embodiment, the trailing time period is six months, although it may be shorter or longer. According to another embodiment, the trailing time period is one year, although it may be shorter or longer. The trailing time period may be predetermined or preprogrammed, or may be determined in whole or in part based on user input received via the user interface.

According to an embodiment, method of calculating mortality reveals changes in mortality risk over a period of time since a triggering event. The period of time may be any period of time, and the triggering event may be any triggering event, including but not limited to a diagnosis, treatment, and other events. According to an embodiment, the risk of non-survival is calculated by representing the number of deaths as a percentage of number of survivors in each time period. This view of data reveals what time periods in the total time period since the triggering event are the most dangerous.

According to one embodiment, the data for the mortality risk graph is calculated using the following equation:

$$Mr = D(time) / S \qquad\qquad (Eq. 1)$$

where Mr is mortality risk, D is the number of deaths during the trailing time period (time), and S is the number of survivors at the beginning of the time period.

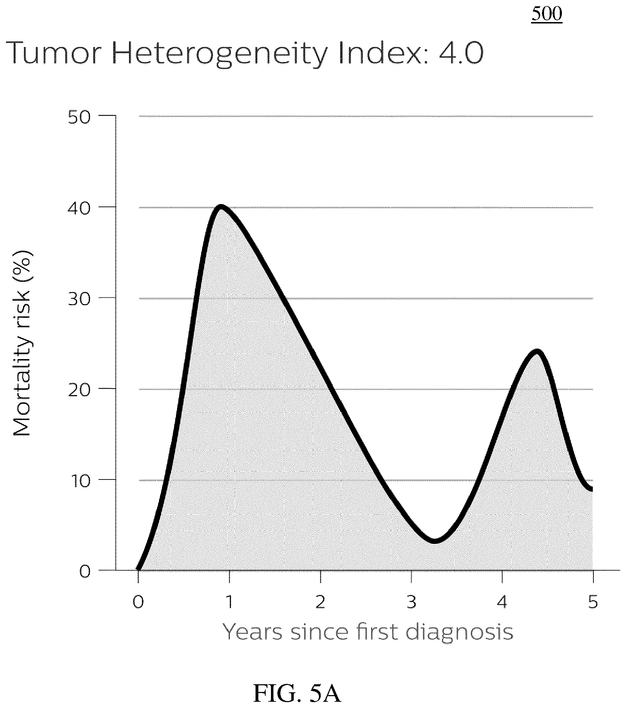

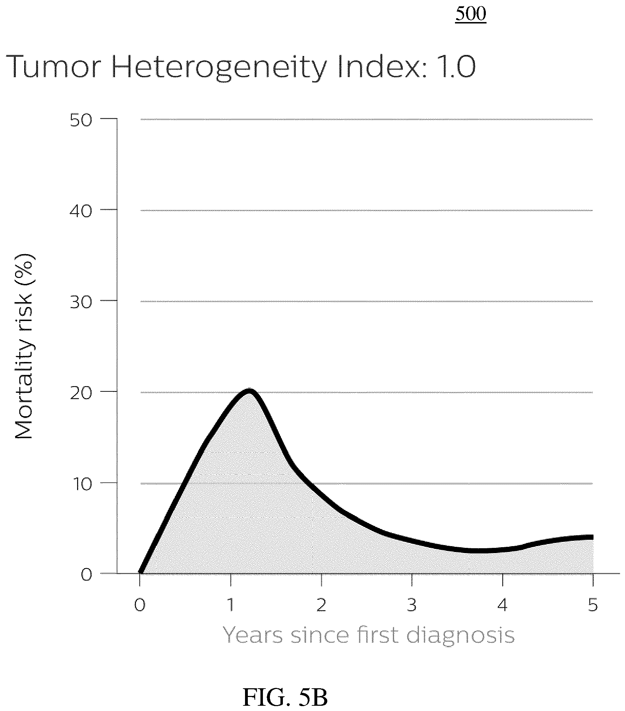

Referring to FIGS. 5A and 5B are mortality risk graphs 500. Each mortality risk graph comprises survival statistics for five years since initial diagnosis, although as described herein this is a non-limiting option. The timeframe may be longer or shorter, and the starting point may be initial diagnosis, treatment, or any other determined starting event. Referring to FIG. 5A, the graph comprises, in this embodiment, a first historical cohort with a particular determined tumor heterogeneity index of 4.0, which indicates a higher level of heterogeneity of cells within the sampled tumor. Referring to FIG. 5B, the graph comprises, in this embodiment, a first historical cohort with a particular determined tumor heterogeneity index of 1.0, which indicates a lower level of heterogeneity of cells within the sampled tumor.

According to another embodiment, the visual representation comprises a survival probability graph. The survival probability graph includes a plot of survival probability over the first time period. The survival probability is calculated for each of a plurality of time points over the first time period by dividing a total number of survivors of the first historical cohort at the end of the first time period by a total number of survivors of the first historical cohort at the time point being calculated.

According to an embodiment, this view focuses on positive aspects of moving along the survival probability timeline. It illustrates the change in prospects of patients that have already moved forward from diagnosis time (or another triggering event). According to an embodiment, the probability of survival till the end of the time period (such as five years) is calculated by comparing the number of overall survivors of the total time interval to the number of survivors at each represented time point. This view of data, when combined with marker representing time that passed since patient diagnosis, may help in visualizing the hope in the upcoming period.

According to one embodiment, the data for the survival probability graph is calculated using the following equation:

$$Probability\ of\ survival(time) = S(time) / S(moment) \qquad\qquad (Eq. 2)$$

where S(time) is the number of survivors at the end of the total time period (time) and S(moment) is the total number of survivors at the moment in time along the graph.

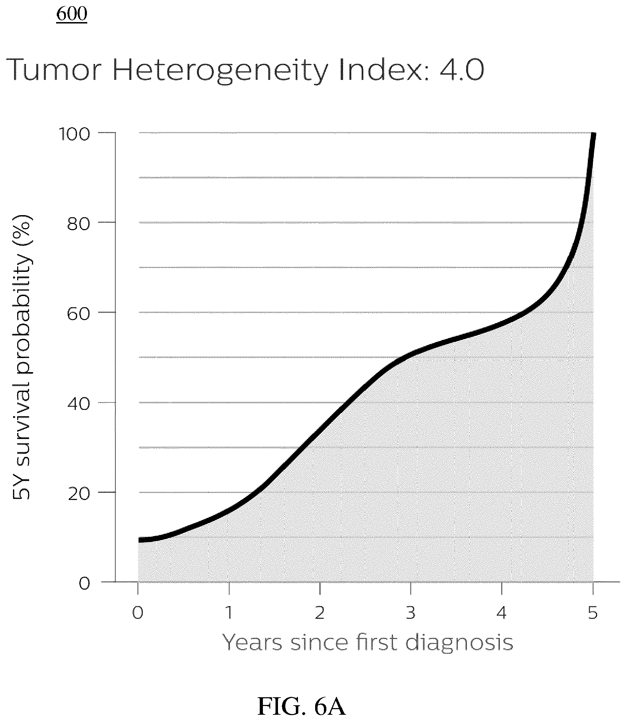

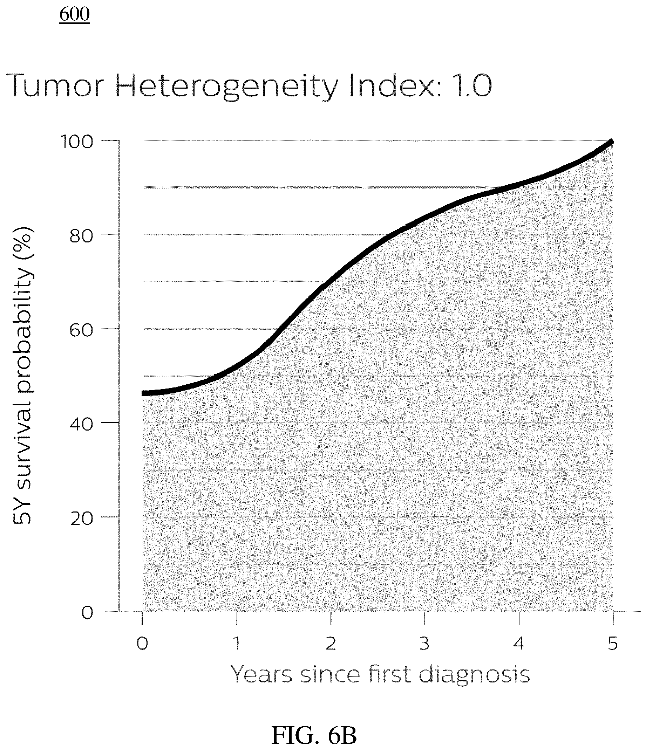

Referring to FIGS. 6A and 6B are survival probability graphs 600. Each survival probability graph comprises survival statistics for five years since initial diagnosis, although as described herein this is a non-limiting option. The timeframe may be longer or shorter, and the starting point may be initial diagnosis, treatment, or any other determined starting event. Referring to FIG. 6A, the graph comprises, in this embodiment, a first historical cohort with a particular determined tumor heterogeneity index of 4.0, which indicates a higher level of heterogeneity of cells within the sampled tumor. Referring to FIG. 6B, the graph comprises, in this embodiment, a first historical cohort with a particular determined tumor heterogeneity index of 1.0, which indicates a lower level of heterogeneity of cells within the sampled tumor.

At step 160 of the method, a user provides input about the displayed one or more visualizations via the user interface of the survival statistics analysis system 200. The input can be any input, including but not limited to a command to modify a visualization, to add a visualization, to remove a visualization, and/or any other input or command. For example, the user interface may be configured to allow a user to navigate between two or more visualizations. The user interface may be configured to display one, two, or more visualizations at one time. The user may be a healthcare professional, the patient, and/or any other user.

Thus, at step 170 of the method, the survival statistics analysis system regenerates one or more visualizations based on the received input, such as returning to a previous step of the method and generating the visualization based on the input. Alternatively, the system may modify a visualization based on the received input. As just one example, the user may adjust one or more of the first historical cohort, the first time period, and a trailing time period for one or more visualizations. This will necessitate that the system recalculate and/or regenerate the visualizations, which can then be displayed via the user interface. Many other modifications are possible.

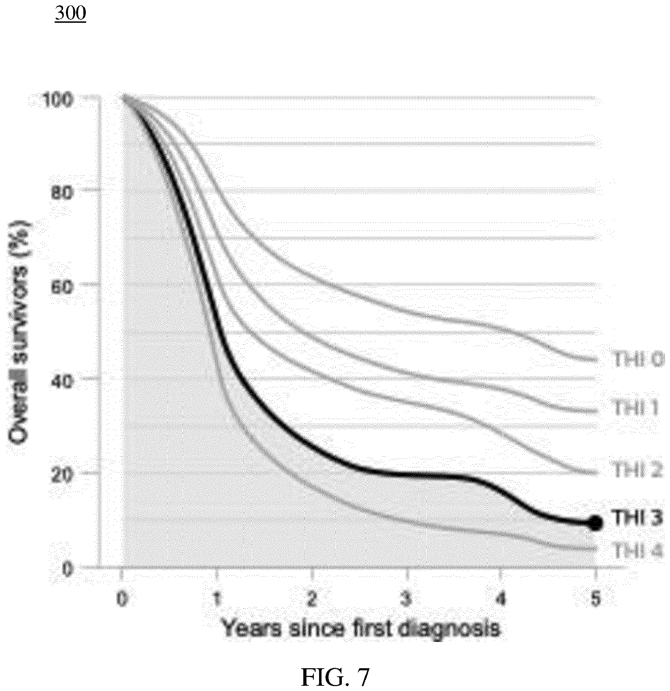

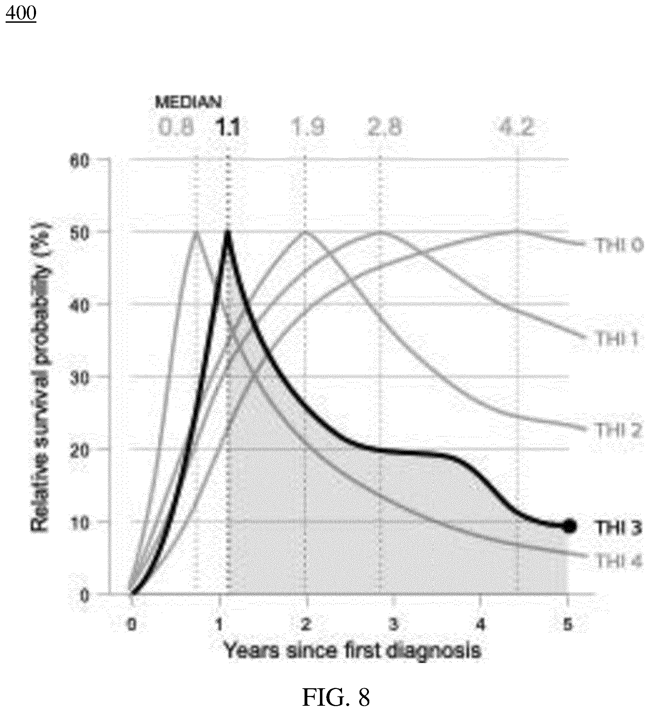

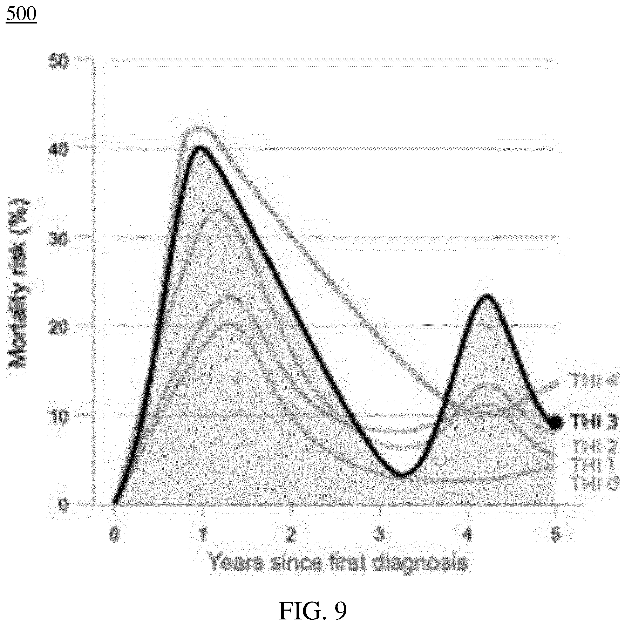

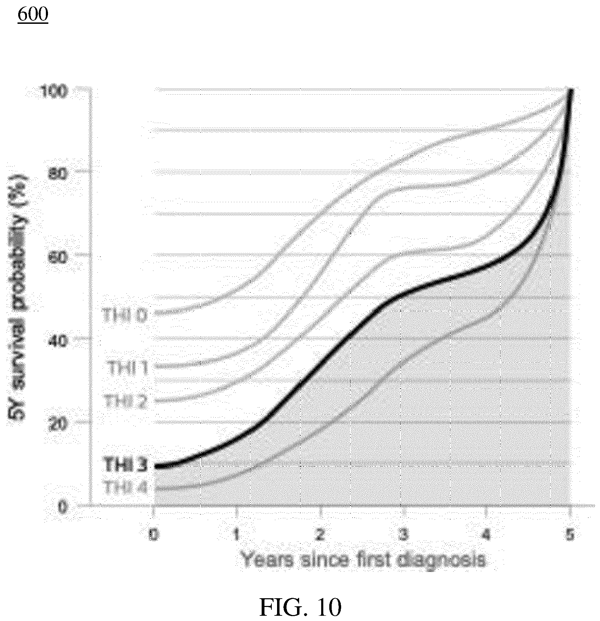

According to an embodiment, any one of the graphs described above or otherwise envisioned herein may comprise a plurality of plots, each calculated with a different historical cohort. For example, the different survival function plots in graph 300 in FIG. 7 each comprise a different historical cohort each having a different tumor heterogeneity index (THI). As another example, the different median survival graphs 400 in FIG. 8 each comprise a different historical cohort each having a different THI. As another example, the different mortality risk graphs 500 in FIG. 9 each comprise a different historical cohort each having a different THI. As another example, the different survival probability graphs 600 in FIG. 10 each comprise a different historical cohort each having a different THI.

Referring to FIG. 2 is a schematic representation of a survival statistics analysis system 200. System 200 may be any of the systems described or otherwise envisioned herein, and may comprise any of the components described or otherwise envisioned herein. It will be understood that FIG. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, the electronic medical record system 270 is an electronic medical records database from which the information about the patient, and/or the reference survival dataset, may be obtained or received. The electronic medical records database may be a local or remote database and is in direct and/or indirect communication with the survival statistics analysis system 200. Thus, according to an embodiment, the system comprises an electronic medical record database or system 270.

According to an embodiment, storage 260 of system 200 may store one or more algorithms, modules, and/or instructions to carry out one or more functions or steps of the methods described or otherwise envisioned herein. For example, the system may comprise, among other instructions or data, patient information 262, reference dataset 263, calculation instructions 264, generation instructions 265, and/or reporting instructions 266.

According to an embodiment, patient information 262 is any information about the patient that the survival statistics analysis system can or may utilize for analysis as described or otherwise envisioned herein. According to an embodiment, the patient information comprises one or more of demographic information about the patient, a diagnosis for the patient, medical history of the patient, and/or any other information. For example, demographic information may comprise information about the patient such as name, age, body mass index (BMI), and any other demographic information. The diagnosis for the patient may be any information about a medical diagnosis for the patient, historical and/or current. The medical history of the patient may be any historical admittance or discharge information, historical treatment information, historical diagnosis information, historical exam or imaging information, and/or any other information. The patient information may be stored in storage 260 of system 200, and/or may be stored in the EMR database or system 270, among other options.

According to an embodiment, the reference survival dataset 263 comprises information about one or more historical patients, which may be collected, filtered, or otherwise combined into one or more patient cohorts. A patient cohort may be, for example, a set of a plurality of historical patients for which survival data is available for at least the first time period. The patient cohort may be comprised of historical patients that have the same diagnosis, conditions, treatment, or other similarities to the patient for which the one or more survival statistics are being calculated. The reference survival dataset 263 may be stored in storage 260 of system 200, and/or may be stored in the EMR database or system 270, among other options.

According to an embodiment, calculation instructions 264 direct the system to calculate one or more survival statistics for the patient. The system may calculate the one or more survival statistics for the patient on demand or as a result of programming or other automation. The one or more survival statistics for the patient are calculated pursuant to any of the methods described or otherwise envisioned herein. For example, described herein are several different non-limiting methods and examples for calculating the one or more survival statistics for the patient. These and many other methods may be utilized by the system.

According to an embodiment, generation instructions 265 direct the system to generate one or more visual representations of the calculated one or more survival statistics. This may be performed immediately after the one or more survival statistics are calculated, or may be performed using stored survival statistics. The system may generate the one or more visual representations of the calculated one or more survival statistics on demand or as a result of programming or other automation. According to an embodiment, the survival statistics analysis system generates the one or more visual representations in response to a user input. For example, a physician or other healthcare professional may request the information. Alternatively, the survival statistics analysis system may be partially or entirely automated, such that the one or more visual representations are automatically calculated. For example, the system may be a standalone system or a component of a medical data or analysis system, which may comprise a user interface that displays information about a patient. This information may comprise one or more of the survival statistics and graphs described or otherwise envisioned herein.

According to an embodiment, reporting instructions 266 direct the system to provide, via a user interface, the generated one or more visual representations of the calculated one or more survival statistics. For example, a visual representation may be displayed to a medical professional or other user, including the patient, via the user interface of the system. The generated one or more visual representations may be provided to a user via any mechanism for display, visualization, or otherwise providing information via a user interface. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands. As just one non-limiting example, the user interface may be a component of a patient monitoring system.

Accordingly, within the context of the disclosure herein, aspects of the embodiments may take the form of a computer program product embodied in one or more non-transitory computer readable media having computer readable program code embodied thereon. Thus, according to one embodiment is a non-transitory computer-readable storage medium comprising computer program code instructions which, when executed by a processor, enables the processor to carry out a method including: (i) receiving information about the patient, comprising at least a patient diagnosis and a date of diagnosis; (ii) calculating, using the patient information and a reference survival dataset, one or more survival statistics for the patient for a first time period relative to a first historical cohort of patients; (iii) generating a visual representation of the calculated one or more survival statistics; and (iv) providing, via a user interface of the system, the generated visual representation. The program code may perform entirely on a user's computer, partly on a user's computer, as a stand-alone software package, partly on a user's computer and partly on a remote computer, or entirely on a remote computer or server.

According to an embodiment, the survival statistics analysis system is configured to process many thousands or millions of datapoints in the calculation of the one or more survival statistics for the patient for the first time period, and in the generation of the one or more visualizations of the calculated one or more survival statistics for the patient, and in the display of the generated one or more visualizations. For example, calculating statistics using data from 100s or 1000s of patients in the historical cohort, and then plotting those calculated statistics, requires processing of millions of datapoints. This requires millions or billions of calculations, which a human mind could not perform in a lifetime.

In addition, the survival statistics analysis system can be configured to continually receive new data for the reference survival dataset. The system can then update the calculations, the visualizations, and the display. This requires the analysis of thousands or millions of datapoints on a continual basis to optimize the display, requiring a volume of calculation and analysis that a human brain cannot accomplish in a lifetime.

By providing improved visualization of survival and mortality statistics, this novel survival statistics analysis system has an enormous positive effect on patient care compared to prior art systems. Improved understanding of survival and mortality statistics, facilitated by the novel visualizations provided herein, can foster improved patient wellbeing and care, among other improvements.

The present invention may be a system, a method, and/or a computer program product. The computer program product may include a non-transitory computer readable storage medium (or media) having computer readable program instructions thereon for causing a system or processor to carry out aspects of the present invention. The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any combination of the foregoing, among other possibilities. Computer readable program instructions described herein can be downloaded to respective computing/processing devices from a computer readable storage medium or to an external computer or external storage device via a network, for example, the internet, a local area network, and/or a wireless network. Aspects of the present invention are described herein with reference to flowchart illustrations and/or block diagrams of methods, apparatus, systems, and computer program products according to embodiments of the invention. It will be understood that each block of the flowchart illustrations and/or block diagrams, and combinations of blocks in the flowchart illustrations and/or block diagrams, can be implemented by computer readable program instructions.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

**Claims**

1. A method (100) for providing, using a survival statistics analysis system (200) a visual representation of survival for a patient, comprising:

   receiving (120) information about the patient, comprising at least a patient diagnosis;
   calculating (130), using the patient information and a reference survival dataset, one or more survival statistics for the patient for a first time period relative to a first historical cohort of patients;
   generating (140) a visual representation of the calculated one or more survival statistics; and
   providing (150), via a user interface of the system, the generated visual representation, wherein the one or more survival statistics comprises one or more of:

   1. a survival function graph, the survival function graph comprising a single graph with both: (i) a plot of a Kaplan-Meier estimate for the first historical cohort for the first time period; and (ii) a plot of trailing mortality, comprising a percentage of mortality of the first historical cohort for a trailing time period, the trailing time period being a windowed segment of the first time period;
   2. a median survival graph, the median survival graph comprising a single graph with (i) an identification of a median survival time for the first historical cohort for the first time period; and (ii) a plot of a percentage of mortality of the first historical cohort for a pre-median survival time period; and (iii) a plot of a percentage of survival of the first historical cohort for a post-median survival time period;
   3. a mortality risk graph, the mortality risk graph comprising a plot of trailing mortality, comprising a percentage of mortality, for a trailing time period, of the first historical cohort relative to a number of survivors of the first historical cohort at a beginning of the trailing time period, the trailing time period being a windowed segment of the first time period; and
   4. a survival probability graph, the survival probability graph comprising a plot of survival probability over the first time period, wherein the survival probability is calculated for each of a plurality of time points over the first time period by dividing a total number of survivors of the first historical cohort at the end of the first time period by a total number of survivors of the first historical cohort at the time point being calculated.

2. The method of claim 1, wherein the first time period is 5 years.

3. The method of claim 1, wherein the trailing time period is 6 months.

4. The method of claim 1, wherein the survival function graph further comprises a final survivor probability estimate for the first time period.

5. The method of claim 1, wherein the median survival graph further comprises a final survivor probability estimate for the first time period.

6. The method of claim 1, wherein providing the generated visual representation comprises providing two or more of: (i) the survival function graph; (ii) the median survival graph; (iii) the mortality risk graph; and (iv) the survival probability graph.

7.  The method of claim 6, wherein the user interface is configured to allow a user to navigate between the two or more graphs.

8.  The method of claim 6, wherein the user interface is configured to display two or more of the graphs at once.

9.  The method of claim 1, wherein each graph is calculated using each of a plurality of different historical cohorts of patients, each of the plurality of different historical cohorts of patients comprising a different relevant condition for the patients in that respective historical cohort.

10. A system (200) for providing a visual representation of survival for a patient, comprising:

    patient information (262) comprising at least a patient diagnosis;
    a reference survival dataset (263);
    a processor (220) configured to: (i) receive information about the patient, comprising at least a patient diagnosis and a date of diagnosis; (ii) calculate, using the patient information and a reference survival dataset, one or more survival statistics for the patient for a first time period relative to a first historical cohort of patients; and (iii) generate a visual representation of the calculated one or more survival statistics; and
    a user interface (240) configured to provide the generated visual representation, wherein the generated visual representation comprises one or more of:

    1. a survival function graph, the survival function graph comprising a single graph with both: (i) a plot of a Kaplan-Meier estimate for the first historical cohort for the first time period; and (ii) a plot of trailing mortality, comprising a percentage of mortality of the first historical cohort for a trailing time period, the trailing time period being a windowed segment of the first time period;
    2. a median survival graph, the median survival graph comprising a single graph with (i) an identification of a median survival time for the first historical cohort for the first time period; and (ii) a plot of a percentage of mortality of the first historical cohort for a pre-median survival time period; and (iii) a plot of a percentage of survival of the first historical cohort for a post-median survival time period;
    3. a mortality risk graph, the mortality risk graph comprising a plot of trailing mortality, comprising a percentage of mortality, for a trailing time period, of the first historical cohort relative to a number of survivors of the first historical cohort at a beginning of the trailing time period, the trailing time period being a windowed segment of the first time period; and
    4. a survival probability graph, the survival probability graph comprising a plot of survival probability over the first time period, wherein the survival probability is calculated for each of a plurality of time points over the first time period by dividing a total number of survivors of the first historical cohort at the end of the first time period by a total number of survivors of the first historical cohort at the time point being calculated.

11. The system of claim 10, wherein the first time period is 5 years.

12. The system of claim 10, wherein the trailing time period is 6 months.

13. The system of claim 10, wherein the survival function graph further comprises a final survivor probability estimate within the first time period.

14. The system of claim 10, wherein the median survival graph further comprises a final survivor probability estimate for the first time period.

15. The system of claim 10, wherein the user interface is configured to provide two or more of: (i) the survival function graph; (ii) the median survival graph; (iii) the mortality risk graph; and (iv) the survival probability graph.

100

| Provide a survival statistics analysis system |
| :---: |
| 110 |

⬇

| Receive patient information |
| :---: |
| 120 |

⬇

| Calculate one or more survival statistics for the patient |
| :---: |
| 130 |

⬇

| Generate one or more visual representations of the calculated patient statistics |
| :---: |
| 140 |

⬇

| Provide the generated visual representations to a user |
| :---: |
| 150 |

⬇

| Receive an input from a user |
| :---: |
| 160 |

⬇

| Regenerate or modify a visual representation or display based on the user input |
| :---: |
| 170 |

FIG. 1

270

Electronic medical
record system

200

220  Processor

230  Memory

240  User Interface

212  System Bus

Communication
Interface

250

261  Operating System

262  Patent Information

263  Reference Survival
Dataset

264  Calculation
Instructions

265  Generation
Instructions

266  Reporting
Instructions

Storage

260

FIG. 2

FIG. 3A

FIG. 3B

FIG. 4A

400

Tumor Heterogeneity Index: 1.0

410

median
**4.2** years

440

5 year
survival
**47**%

420

430

450

Relative survival probability (%)

6 month trailing mortality (%)

Years since first diagnosis

FIG. 4B

FIG. 5A

<u>500</u>

## Tumor Heterogeneity Index: 1.0

FIG. 5B

600

Tumor Heterogeneity Index: 4.0

FIG. 6A

600

Tumor Heterogeneity Index: 1.0

FIG. 6B

300

FIG. 7

400

FIG. 8

<u>500</u>

FIG. 9

600

FIG. 10

Europäisches
Patentamt

European
Patent Office

Office européen
des brevets

# EUROPEAN SEARCH REPORT

Application Number

**EP 22 19 4572**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/319908 A1 (LEFKOFSKY HAILEY [US] ET AL) 14 October 2021 (2021-10-14) * abstract; figures 1,2, 15-20 * * paragraph [0049] – paragraph [0083] * * paragraph [0106] – paragraph [0116] * * paragraph [0156] * ----- | 1-15 | INV. G16H50/30 G16H50/70 G16H15/00 |
| | | | TECHNICAL FIELDS SEARCHED (IPC) |
| | | | G16H |

The present search report has been drawn up for all claims

| Place of search | Date of completion of the search | Examiner |
|---|---|---|
| Munich | 30 January 2023 | Menschner, Philipp |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 19 4572

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2023

| Patent document cited in search report | | Publication date | Patent family member(s) | | Publication date |
|---|---|---|---|---|---|
| US 2021319908 | A1 | 14-10-2021 | AU | 2019418813 A1 | 22-07-2021 |
| | | | CA | 3125449 A1 | 09-07-2020 |
| | | | EP | 3906564 A1 | 10-11-2021 |
| | | | JP | 2022516172 A | 24-02-2022 |
| | | | US | 2020211716 A1 | 02-07-2020 |
| | | | US | 2021125730 A1 | 29-04-2021 |
| | | | US | 2021125731 A1 | 29-04-2021 |
| | | | US | 2021319908 A1 | 14-10-2021 |
| | | | US | 2021350937 A1 | 11-11-2021 |
| | | | WO | 2020142551 A1 | 09-07-2020 |

EPO FORM P0459

For more details about this annex : see Official Journal of the European Patent Office, No. 12/82